# EUROPÄISCHE PATENTANMELDUNG

(11) **EP 3 130 358 A1**
(43) Veröffentlichungstag der Anmeldung: **15.02.2017**
(21) Anmeldenummer: 15181083.5
(22) Anmeldetag: 14.08.2015
(51) Int. Cl.: A61L 2/00, A61L 2/22

(54) **VERFAHREN UND SYSTEM ZUR ENTKEIMUNG VON MEDIZINISCHEN WERKZEUGEN UND KORPERTEILEN**

(71) Anmelder: Siefer, Stephan, 59199 Bönen (DE); Klein, Klaus, 85055 Ingolstadt (DE)
(72) Erfinder: KLEIN, Klaus, 85055 Ingolstadt (DE)
(74) Vertreter: Stenger Watzke Ring

(57) **Zusammenfassung**

Die Erfindung betrifft ein Verfahren zur Entkeimung von Räumen oder Bereichen hiervon, bei dem ein Desinfektionsmittel verwendet wird, welches ein Oxidationsmittel und ein Tensid aufweist, bei dem aus dem Desinfektionsmittel ein schwebfähiges Aerosol erzeugt und kontinuierlich in den zu entkeimenden Raum eingebracht wird, bei dem die Gesamtkonzentration des Desinfektionsmittels im Aerosol eingestellt wird, wobei jeweils ein maximaler Grenzwert für die Einzelkonzentration des Oxidationsmittels und des Tensids im Aerosol vorgegeben wird.

## Beschreibung

Die Erfindung betrifft ein Verfahren zur Entkeimung von Räumen, ein Desinfektionsmittel zum Einsatz im erfindungsgemäßen Verfahren, eine Vorrichtung zur Durchführung des erfindungsgemäßen Verfahrens sowie ein System zur Entkeimung von Räumen.

Seit der Entdeckung der Übertragbarkeit von Krankheiten durch Keime im 19. Jahrhundert nimmt die Bedeutung der Hygiene im Allgemeinen und der Keimbekämpfung im Speziellen stetig zu. Insbesondere in hygienisch besonders sensiblen Umfeldern, wie zum Beispiel Krankenhäusern und Arztpraxen, aber auch in der Lebensmittelindustrie ist eine wirksame Keimbekämpfung von essentieller Wichtigkeit. Die Raumentkeimung hat sich diesbezüglich in der Praxis als besonders effektiv erwiesen.

Aus dem Stand der Technik sind zum Beispiel Verfahren bekannt, bei denen eine Raumentkeimung dadurch durchgeführt wird, dass der zu entkeimende Raum verschlossen, insbesondere versiegelt, und anschließend begast wird. Nach einer vorgebbaren Einwirkzeit von zum Beispiel zwei Stunden gilt der Raum als entkeimt. Aufgrund der Aggressivität des verwendeten Desinfektionsmittels, welches zumeist vergleichsweise hohe Konzentrationen an chlorhaltigen Verbindungen oder Ozon aufweist, dürfen sich Menschen während der Einwirkzeit nicht in diesem Raum aufhalten. Nach Abschluss der Entkeimung ist der Raum durchzulüften, um das eingesetzte Gas wieder zu entfernen. Alsdann kann der Raum wieder von Menschen betreten werden.

Nachteilig bei diesem Vorgehen ist, dass mit dem Lüften sowie mit dem Eintritt der Menschen in den Raum eine Wiederverkeimung stattfindet. Diese Wiederverkeimung geht derart schnell vonstatten, dass binnen kürzester Zeit der Keimstatus wiederhergestellt ist, wie er vor der Entkeimung bestand.

Zur Entkeimung von Luft ist es ferner bekannt, die zu entkeimende Luft mit elektromagnetischer Strahlung im ultravioletten Bereich des Spektrums zu bestrahlen. Hierzu wird Luft von einer entsprechenden Vorrichtung angesaugt, bestrahlt und wieder in den Raum eingeleitet. Eine Entkeimung der im Raum befindlichen Oberflächen, von insbesondere Wänden, Möbeln und dergleichen findet nicht statt, so dass eine Wiederverkeimung durch Kontakt der Luft mit besagten Oberflächen nicht verhindert werden kann.

Insbesondere zur Oberflächenentkeimung ist es aus dem Stand der Technik ferner bekannt, diese lokal mit einem Desinfektionsmittel zu besprühen und händisch, insbesondere durch wischen, zu reinigen. Es kommen zu diesem Zweck Tücher oder ähnliche Reinigungshilfen zum Einsatz, die mit besagtem Desinfektionsmittel besprüht werden müssen, oder bereits vorbehandelt sind. Es folgt ein Abwischen der zu reinigenden Oberflächen. Von Nachteil auch dieses Verfahrens ist, dass eine Wiederverkeimung stattfindet. Darüber hinaus ist die Qualität der Entkeimung von der Sorgfältigkeit beim Wischen abhängig. Hinzukommt, dass mit Hilfe des Wischens nicht alle Oberflächen gereinigt werden, sei es, dass diese nur schwer zugänglich sind oder dass sie überhaupt nicht abgewischt werden können, wie zum Beispiel aus Stoff gefertigte Gardinen, Bettwäsche, Tischdecken oder dergleichen.

Es ist ferner aus der DE 10 2010 020 508 A1 eine Injektionsvorrichtung bekannt, die zur Einleitung von Desinfektionsmitteln und/oder Duftstoffen in einen Volumenstrom eines Leitungssystems geeignet ist. Hierbei werden die Mittel, vorzugsweise in flüssiger Form, in das Leitungssystems eingedüst. Hierzu ist ein Injektionskopf vorgesehen, der insbesondere als Düse und besonders bevorzugt als Hohlkegeldüse ausgebildet sein kann. Die Eindüsung erfolgt hierbei intervallweise. Nachteilig hieran ist, dass in den Intervallpausen, in denen keinerlei Begasung stattfindet, eine Wiederverkeimung auftritt. Darüber hinaus hat sich herausgestellt, dass die sich aus der Kombination von Injektionsvorrichtung und Desinfektionsmittel ergebende Desinfektionsleistung insbesondere aufgrund der mangelnden Verteilbarkeit im Raum keine ausreichende Desinfektion gewährleisten kann.

Es ist daher die Aufgabe der Erfindung, ein Verfahren sowie ein System zur Entkeimung von Räumen bereitzustellen, mit welchen eine Entkeimung erreicht und eine Wiederverkeimung verhindert wird.

Zur Lösung dieser Aufgabe wird ein Verfahren zur Entkeimung von Räumen oder Bereichen hiervon, bei dem ein Desinfektionsmittel verwendet wird, welches ein Oxidationsmittel und ein Tensid aufweist, bei dem aus dem Desinfektionsmittel ein schwebfähiges Aerosol erzeugt und kontinuierlich in den zu entkeimenden Raum eingebracht wird, bei dem die Gesamtkonzentration des Desinfektionsmittels im Aerosol eingestellt wird, wobei jeweils ein maximaler Grenzwert für die Einzelkonzentration des Oxidationsmittels und des Tensids im Aerosol vorgegeben wird.

Durch die erfindungsgemäße kontinuierliche Verfahrensführung wird eine dauerhaft sichere Entkeimung erreicht und eine Wiederverkeimung wirksam vermieden. Kontinuierlich im Sinne der Erfindung bezeichnet hierbei die zeitlich beliebig lange Durchführung des Verfahrens ohne dass eine bestimmungsgemäße Nutzung des zu entkeimenden Raumes oder des Bereiches hiervon unterbrochen werden müsste. Erfindungsgemäß wird hiermit eine kontinuierliche Abtötung von krankheitserregenden, gesundheitsschädlichen oder ähnlichen Keimen erreicht und eine Wiederverkeimung für die Dauer des angewendeten Verfahrens verhindert. Zu besagten Keimen zählen insbesondere Bakterien, Viren, Pilze, Sporen, Feinstäube und ähnliches.

Eine erfolgreiche kontinuierliche Verfahrensführung zur Entkeimung hängt erfindungsgemäß vom synergetischen Zusammenwirken einzelner erfindungswesentlicher Merkmale ab. So ist es zum einen erforderlich, die Einzelkonzentrationen der verwendeten Komponenten des Desinfektionsmittels unterhalb eines jeweils vorgebbaren Grenzwertes zu halten. Hierdurch wird eine Verfahrensführung ohne Beeinträchtigung der bestimmungsgemäßen Nutzung des zu entkeimenden Raumes ermöglicht. Das erfindungsgemäße Verfahren ist in einer Vielzahl von Bereichen Einsetzbar. Insbesondere im Gesundheitssektor, dort bevorzugt in Krankenhäusern, Pflegeheimen, Arztpraxen und dergleichen, in der Nahrungsmittelindustrie, dort insbesondere in der Viehhaltung, der weiterverarbeitenden Industrie, insbesondere Metzgereien, Bäckereien und dergleichen, und der Lagerhaltung. Anwendungen im privaten Sektor sind ebenfalls denkbar. Die vorzugebenden Grenzwerte können sich aus Erfahrungswerten im jeweiligen Sektor ergeben. Alternativ können sie sich auch durch gesetzliche Regelungen ergeben. Insbesondere in zu entkeimenden Bereichen, in welchen im bestimmungsgemäßen Fall der Nutzung Menschen arbeiten, ist der jeweilige erfindungsgemäße Grenzwert vorzugsweise ein gesetzlich vorgeschriebener Grenzwert, der sicherstellt, dass die Verwendung der einzelnen Komponenten im Desinfektionsmittel und damit das Desinfektionsmittel als solches für den Menschen keinerlei gesundheitsschädliche Wirkung entfalten. Vorzugsweise ist der jeweilige Grenzwert hierbei durch den gesetzlichen Arbeitsplatzgrenzwert (AGW; früher Maximale Arbeitsplatzkonzentration - MAK) für die Komponenten des Desinfektionsmittels gegeben. In Bereichen die mehreren gesetzlichen Regelungen unterworfen sind wird vorzugsweise der jeweils niedrigere Grenzwert eingehalten. Dies ist insbesondere in der Lebensmittelindustrie der Fall, wo sowohl Arbeitsplatzgrenzwerte als auch Grenzwerte der Lebensmittelhygiene-Verordnung (LMHV) gelten. Erfindungsgemäß wird die Gesamtkonzentration des Desinfektionsmittels im Aerosol so gewählt, dass die Einzelkonzentration sowohl des Oxidationsmittel als auch des Tensids unterhalb eines solchen Grenzwertes liegen. Bei aus dem Stand der Technik bekannten Verfahren zur Raumentkeimung wird typischerweise von Konzentrationen Gebrauch gemacht, die deutlich oberhalb der vorbeschriebenen Grenzwerte liegen, um eine Entkeimung überhaupt realisieren zu können. Die vorherrschende Meinung im Stand der Technik ist daher die, dass für eine ordnungsgemäße Entkeimung vergleichsweise hohe Konzentrationen an Wirkstoffen eingesetzt werden müssen, die für Menschen, Tiere, Lebensmittel und dergleichen schädlich sind. Die Erfindung schlägt hier in bewusster Abkehr vom Stand der Technik einen neuen Weg ein.

Der Anmelder hat überraschend herausgefunden, dass ein Desinfektionsmittel wenigstens bestehend aus einem Oxidationsmittel und einem Tensid in einer vorbeschriebenen Konzentration im Aerosol eine niemals für möglich gehaltene Wirksamkeit gegenüber Keimen aufweist. Vorzugsweise wird eine erfindungsgemäße Entkeimung in überraschender Weise bereits bei vergleichsweise geringeren Gesamtkonzentrationen erreicht. Vorzugsweise ist bereits eine Gesamtkonzentration von 0,01 ml bis 0,05 ml Desinfektionsmittel/m³ Raumluft für eine erfindungsgemäße Raumentkeimung ausreichend. Als besonders vorteilhaft hat sich hierbei eine Konzentration von 0,02 ml Desinfektionsmittel/m³ Raumluft erwiesen. Die verwendeten Gesamtkonzentrationen liegen damit deutlich unterhalb der AGW von für die Bildung des erfindungsgemäßen Desinfektionsmittels geeigneten Oxidationsmitteln wie insbesondere, Natriumhypochlorit (0,5 ml/m³), Wasserstoffperoxid (0,5 ml/m³), Ozon (0,1 ml/m³) oder Peroxyessigsäure (10 ml/m³). Gleichermaßen liegen die Gesamtkonzentrationen des Desinfektionsmittels im Aerosol deutlich unterhalb der für Stäube im Allgemeinen und für Tenside im speziellen geltenden AGW von ca. 10 ml/m³.

Es hat sich ferner herausgestellt, dass bereits eine Volumenkonzentration des Desinfektionsmittels deutlich unterhalb der vorgenannten 0,01 ml/m³ - 0,05 ml/m³ Raumluft für eine vollstände Entkeimung ausreichend ist. Dementsprechend ist es bevorzugt, Desinfektionsmittel mit einer Konzentration zwischen 0,1 und 0,5 µl pro m³ Raumluft einzusetzen

Das zur Entkeimung verwendete Aerosol ist damit nicht nur für Menschen gesundheitlich unbedenklich sondern darüber hinaus wirksam gegenüber Keimen und beeinträchtigt in keiner Weise die bestimmungsgemäße Nutzung des zu entkeimenden Raumes, wodurch das erfindungsgemäße Verfahren durch seine kontinuierliche Anwendung für eine initiale Entkeimung sorgt und darüber hinaus eine Wiederverkeimung in vorteilhafter Weise verhindert. Insbesondere im Gesundheitswesen kann das erfindungsgemäße Verfahren damit ohne Rücksichtnahme von im Raum anwesenden Menschen kontinuierlich durchgeführt werden. Eine Wiederverkeimung, wie sie bei den aus dem Stand der Technik bekannten Verfahren auftritt, kann mit der erfindungsgemäßen Ausgestaltung vorteilhafterweise vermieden werden, so dass in dem zu entkeimenden Raum auch bei Einbringung von frischen Keimen in besagten Raum, durch zum Beispiel Lüften, ein konstant niedriges Keimniveau beibehalten werden kann.

Gemäß der Erfindung wird aus dem Desinfektionsmittel ein schwebfähiges Aerosol erzeugt. Im Stand der Technik ist es demgegenüber aufgrund des Einsatzes von Düsen, insbesondere Hohlkegeldüsen bislang nicht möglich, ein schwebfähiges Aerosol zu erzeugen. Erfindungsgemäß wird das Desinfektionsmittel mit Luft gemischt. Luft dient mithin als Trägermedium für das Desinfektionsmittel. Das Desinfektionsmittel kann auf dem Wege des Luftkonvektion in einfacher Weise in dem zu entkeimenden Raum oder in auch nur in Teilen hiervon verteilt werden. Vorteilhafterweise wird hierdurch eine Entkeimung der Luft selbst und sämtlicher Oberflächen im zu entkeimenden Raum erreicht. Insbesondere Oberflächen, die im Stand der Technik in der Regel durch Wischen nur unzureichend entkeimt wurden, können durch die Erfindung nahezu vollständig entkeimt werden. Ferner ist es durch die Verwendung von Luft als Trägermedium nunmehr möglich auch Oberflächen zu entkeimen, die durch bloßes Wischen nicht entkeimt werden können. Dazu zählen insbesondere raue Oberflächen, poröse Oberflächen, texturierte Oberflächen, textile Oberflächen, wie insbesondere Vorhänge, Bettwäsche, Kleidung und dergleichen. Das Aerosol dringt in mikroskopische Strukturen dieser Oberflächen ein und entkeimt diese auch an für herkömmliche Verfahren unzugänglichen Stellen. Vorzugsweise wird Luft in Form von Umgebungsluft verwendet. Alternativ zu oder in Kombination mit der Umgebungsluft kann es vorgesehen sein, ein eigens hergestelltes luftähnliches Gasgemisch zu verwenden. Luftähnlich bedeutet in diesem Zusammenhang, dass das Gasgemisch Stickstoff und Sauerstoff in einem für den Metabolismus von Menschen und/oder Tieren ausreichenden Anteil enthält. Insbesondere Für den Fall, dass die Umgebungsluft nicht oder nicht als solches für die Erzeugung des Aerosols geeignet ist oder aus anderen Gründen nicht als Trägermedium verwendet werden kann, ist dies besonders vorteilhaft.

Schwebfähigkeit des Aerosols ist vorteilhaft, da hierdurch die Verteilbarkeit des Aerosols verbessert wird. Es ist insbesondere möglich, auch entfernte Raumbereiche mit Aerosol zu beaufschlagen, ohne dass das Desinfektionsmittel zuvor zu Boden sinkt. Bei der Erzeugung des Aerosols wird das Desinfektionsmittel gemäß einer bevorzugten Ausgestaltung der Erfindung daher zu feinen Tröpfchen mit einem Durchmesser unterhalb von 10 µm zerstäubt. Vorteilhafterweise kann auf diese Art ein schwebfähiges und stabiles Aerosol erzeugt werden. Es hat sich ferner herausgestellt, dass Tröpfchen mit einem solchen Durchmesser insbesondere bei strukturierten und/oder porösen Oberflächen, wie sie insbesondere bei Textilien vorkommen, aufgrund einer verbesserten Materialgängigkeit zu einer besonders effektiven Entkeimung führen. Zur weiteren Verbesserung der Materialgängigkeit ist es bevorzugt, Tröpfchen mit einem Durchmesser unterhalb von 5 µm und weiter bevorzugt zwischen 1 µm und 3 µm zu erzeugen.

Erfindungsgemäß wird das Aerosol nach dessen Erzeugung kontinuierlich in den zu entkeimenden Raum eingebracht. Dies kann gemäß einer bevorzugten Ausgestaltung der Erfindung direkt und unmittelbar geschehen. Hierzu kann eine für die Durchführung des Verfahrens geeignete Vorrichtung in dem zu entkeimenden Raum bzw. dem zu entkeimenden Raumbereich aufgestellt werden. Vorzugsweise ist eine solche Vorrichtung mobil ausgebildet, um verschiedene Räume oder Raumbereiche je nach Wunsch oder Notwendigkeit zu entkeimen. Vorzugsweise weist die Vorrichtung hierzu Bewegungsmittel auf. Die Bewegungsmittel können bevorzugt als Rollen, Räder, Kufen oder dergleichen ausgebildet sein. Das erfindungsgemäße Verfahren ist vorzugsweise für jede Raumgröße skalierbar. Hierzu wird gemäß einer bevorzugten Ausführungsform der Erfindung die Leistungsfähigkeit der Vorrichtung an die Größe des zu entkeimenden Raumes angepasst. Gemäß einer weiteren, mit der vorgenannten Ausgestaltung kombinierbaren Ausgestaltung der Erfindung wird die Zahl der in einem Raum aufzustellenden Vorrichtungen zum Zwecke der Skalierbarkeit an die Größe des Raumes angepasst. Auf diesem Wege ist es möglich, das erfindungsgemäße Verfahren an jede beliebige Raumgröße anzupassen.

Gemäß einer alternativen Ausgestaltung der Erfindung wird das Aerosol mittelbar in den zu entkeimenden Raum eingebracht. Es ist hierbei nicht notwendig, die Vorrichtung in dem zu entkeimenden Raum aufzustellen. Vielmehr ist diese lediglich leitungstechnisch mit dem zu entkeimenden Raum verbunden. Eine solche Verfahrensführung erlaubt vorteilhafterweise eine räumliche Entkappelung der Aerosolerzeugung von deren Verwendungsort. Hieraus ergibt sich eine ganze Reihe von Vorteilen. Einerseits sind etwaige mit der Aerosolherstellung einhergehende Effekte, wie insbesondere eine als unangenehm empfundene Geräuschentwicklung in dem zu entkeimenden Raum nicht wahrnehmbar. Dies ist insbesondere im Krankenhaus wo typischerweise ruhebedürftige Personen behandelt werden von Vorteil. Andererseits ist die Vorrichtung auch optisch nicht in dem zu entkeimenden Raum präsent, wodurch das Erscheinungsbild des Raumes nicht unangemessen gestört wird. Insbesondere in Arztpraxen, in denen regelmäßig viel Wert auf die Inneneinrichtung gelegt wird, ist eine solche Entkopplung von Vorteil. Vorzugsweise ist die Vorrichtung leitungstechnisch an mehr als nur einen Raum angeschlossen, wodurch mit einer Vorrichtung mehrere Räume entkeimt werden können. Bevorzugt ist die Anordnung der Vorrichtung in einem bereits bestehenden Belüftungssystem eines Gebäudes, welches eine Vielzahl von Räumen miteinander verbindet. Vorzugsweise wird die Vorrichtung an einem Knotenpunkt des Belüftungssystems angeordnet, um möglichste viele Räume gleichermaßen zu entkeimen. Der in einem solchen Belüftungssystem vorliegende Luftstrom umspült dabei die Vorrichtung. Er dient hierbei vorteilhafterweise als Trägermedium für das Desinfektionsmittel im Aerosol.

Die Erfindung betrifft darüber hinaus ein Desinfektionsmittel zum Einsatz im erfindungsgemäßen Verfahren. Dieses zeichnet sich durch eine besondere Wirkstoffkombination aus wenigstens einem Oxidationsmittel und einem Tensid aus.

Der Anmelder hat herausgefunden, dass die erfindungsgemäße Wirkstoffkombination bei der Keimbekämpfung eine synergetische Wirkung entfaltet und somit besonders effektiv ist. Es ist hierdurch möglich, vergleichsweise geringe Konzentrationen an Desinfektionsmittel zur Entkeimung zu verwenden, was eine für den Menschen unschädliche, kontinuierliche Verfahrensführung überhaupt erst ermöglicht.

Ohne auf diesen Mechanismus beschränkt zu sein, wird anmelderseitig vermutet, dass das Oxidationsmittel die äußeren Strukturen des jeweiligen Keims angreift und dem Tensid auf diese Art den Zugang zum Inneren des Keimes ermöglicht, wo dieser seine für den Keim schädliche Wirkung mit vergleichsweise hoher Wirksamkeit entfalten kann.

Gemäß einer bevorzugten Ausgestaltung der Erfindung ist das Oxidationsmittel aus Wasserstoffperoxid (H₂O₂) gebildet. Es zeichnet sich in vorteilhafter Weise durch ein vergleichsweise hohes Standardelektrodenpotential (Redoxpotential unter Standardbedingungen) von 1,78 V aus, womit es zu den starken Oxidationsmitteln gehört. Neben seiner Rolle als Oxidationsmittel ist Wasserstoffperoxid im Bereich der Raumentkeimung besonders vorteilhaft, da es während der Entkeimung zu Wasser (H₂O) reduziert wird, so dass keinerlei schädliche Nebenprodukte entstehen. Ferner neigt Wasserstoffperoxid dazu im Rahmen einer Disproportionierungsreaktion in Wasser und Sauerstoff zu zerfallen. Im Bereich der Raumentkeimung hat sich dieses Verhalten neben der eigentlichen Entkeimung als vorteilhaft erwiesen, da eine sukzessive Anreicherung der Umgebungsluft mit Sauerstoff stattfindet, was von den sich im Raum aufhaltenden Personen im Allgemeinen als angenehm und "frisch" empfunden wird. Hieraus ergibt sich insbesondere mit der Wirksamkeit des Desinfektionsmittels gegenüber Feinstaub mit Bezug auf die Luftauffrischung ein besonders positiver synergetischer Effekt.

Das erfindungsgemäß als Komponente im Desinfektionsmittel eingesetzte Tensid ist gemäß einem bevorzugten Merkmal der Erfindung ein pflanzliches Tensid. Pflanzlich bedeutet in diesem Zusammenhang, dass das Tensid aus pflanzlichen Rohstoffen, insbesondere Fetten gewinnbar ist. Es kann vorzugsweise vorgesehen sein, dass das Desinfektionsmittel mehr als ein Tensid enthält, um eine möglichst breite Entkeimungswirksamkeit zu erreichen. Es hat sich diesbezüglich herausgestellt, dass unterschiedliche Tenside unterschiedlich wirksam gegenüber verschiedenen Arten von Keimen sind. Vorzugsweise ist es hierbei vorgesehen, dass sämtliche im Desinfektionsmittel eingesetzten Tenside aus einer einzigen pflanzlichen Quelle gewinnbar sind. Dies kann vorliegend insbesondere Kokosöl sein. Der Anmelder hat herausgefunden, dass die aus den im Kokosöl enthaltenen Fettsäuren gewinnbaren Tenside, insbesondere die Carboxylate der Palmitin-, Laurin-, Myristin- und Ölsäure, eine besonders hohe Wirksamkeit und ein breites Spektrum bei der Keimbekämpfung aufweisen.

Es hat sich bei Wirksamkeitstests des Desinfektionsmittels gezeigt, dass die Wirksamkeit des Desinfektionsmittels von der Art und der genauen quantitativen Zusammensetzung der Einzelkomponenten des Desinfektionsmittels abhängig ist. Es ist prinzipiell möglich, das erfindungsgemäße Aerosol sowohl aus einem Desinfektionsmittel im festen, als auch im flüssigen Zustand zu erzeugen. Es ist jedoch bevorzugt, das Desinfektionsmittel aufgrund der einfacheren Handhabung als wässrige Lösung auszubilden. Vorzugsweise wird hierzu ausschließlich vollentsalztes Wasser verwendet, um ein möglichst reines Desinfektionsmittel bereitzustellen, und auf diesem Wege mögliche Nebenwirkungen oder Nebenreaktionen mit den Wirkstoffen zu vermeiden. Vorzugsweise besteht das Desinfektionsmittel wenigstens aus 95 Gew.% bis 98 Gew.% vollentsalztem Wasser, 1,5 Gew.% bis 4,9 Gew.% Wasserstoffperoxid und 0,1 Gew.% bis 0,5 Gew.% wenigstens eines Tensids. Gemäß einer besonders vorteilhaften Ausgestaltung der Erfindung besteht das Desinfektionsmittel wenigstens aus 97 Gew.% vollentsalztem Wasser, 2,7 Gew.% Wasserstoffperoxid und 0,3 Gew.% eines aus Kokosöl gewinnbaren Tensidgemisches. Vorgenannte Zusammensetzungen haben sich hinsichtlich der Entkeimungszeit, der Entkeimungswirksamkeit sowie des Entkeimungsspektrums als besonders vorteilhaft erwiesen.

Gemäß einer besonders bevorzugten Ausgestaltung der Erfindung ist es vorgesehen, dem Desinfektionsmittel Ozon als weiteres Oxidationsmittel beizufügen. Verfahrensseitig kann dies entweder nachträglich im Laufe des Entkeimungsverfahrens oder bereits im Vorfeld des Verfahrens durch Bereitstellung eines Ozon enthaltenden Desinfektionsmittels realisiert werden. In der elektrochemischen Spannungsreihe liegt das Ozon mit einem Standardelektrodenpotential von 2,075 V über dem von Wasserstoffperoxid und ist dem entsprechend ein stärkeres Oxidationsmittel. Die in das Desinfektionsmittel eingebrachte Konzentration an Ozon ist im Sinne der erfindungsgemäßen Durchführung des Verfahrens ebenfalls so gewählt, dass diese im Aerosol für Menschen nicht gesundheitsschädlich ist. Es hat sich herausgestellt, dass eine solche Konzentration an Ozon in Verbindung mit dem im Desinfektionsmittel enthaltenen Wasserstoffperoxid das Gesamtredoxpotential auf bis zu 2,86 V erhöht. Es ist durch die vorgenannte Kombination von Oxidationsmitteln möglich, das Redoxpotential über den Wert ihrer Einzelkomponenten zu steigern, so dass das Gesamtpotential in synergetischer Weise das Standardelektrodenpotential von Fluor erreicht.

Die Erfindung betrifft ferner eine Vorrichtung zur Durchführung des erfindungsgemäßen Verfahrens. Erfindungsgemäß verfügt die Vorrichtung über einen Desinfektionsmitteltank zur Bereitstellung eines Desinfektionsmittels. Der Desinfektionsmitteltank ist vorzugsweise mit einem Vorratsbehälter leitungstechnisch verbunden. Der Vorratsbehälter dient der Auffüllung des Desinfektionsmitteltanks. Das Fassungsvermögen des Vorratsbehälters ist an die Größe der Vorrichtung anpassbar. Typischerweise nimmt dieser je nach Größe des Geräts zwischen 10 l und 20 I Desinfektionsmittel auf. Es sind jedoch in Abhängigkeit der/des zu entkeimenden Räume/Raumes auch wesentlich größere Behälter denkbar. Vorzugsweise sind Vorratsbehälter und Desinfektionsmitteltank unter Zwischenschaltung einer Pumpe miteinander verbunden. Die Pumpe kann vorzugsweise als Schlauchpumpe, Membranpumpe oder dergleichen ausgebildet sein. Mittels der Pumpe wird das Desinfektionsmittel vorzugsweise aus dem Vorratsbehälter in den Desinfektionsmitteltank gepumpt. Dies kann vorzugsweise automatisch geschehen. Zu diesem Zweck verfügt der Desinfektionsmitteltank über einen Füllstandssensor. Für die bestimmungsgemäße Verfahrensführung ist es vorrichtungsseitig bevorzugt, dass im Desinfektionsmitteltank eine bestimmte, vorgebbare Füllstandhöhe eingehalten wird. Um diese Füllstandhöhe innerhalb des Desinfektionsmitteltanks einzuhalten, ist vorzugsweise der Füllstandssensor vorgesehen. Dieser ist steuerungstechnisch mit der Pumpe verbunden, so dass aus dem Vorratsbehälter bei Unterschreiten der Füllstandhöhe automatisch Desinfektionsmittel nachgepumpt wird, um den zur Funktionstüchtigkeit insbesondere der Zerstäubungsmembran notwendigen Flüssigkeitsspiegel innerhalb des Desinfektionsmitteltanks einzuhalten.

Die Vorrichtung weist erfindungsgemäß ferner eine mit dem Desinfektionsmitteltank leitungstechnisch verbundene Aerosolerzeugungseinheit zur Erzeugung eines Aerosols aus dem Desinfektionsmittel auf. Gemäß einem bevorzugten Merkmal der Erfindung verfügt die Aerosolerzeugungseinheit über eine Zerstäubungsmembran. Diese ist vorzugsweise derart ausgebildet, dass sie in einer einstellbaren Frequenz oszillieren kann. Vorzugsweise wird die Frequenz in Abhängigkeit der zu erzeugenden Aerosolmenge eingestellt. Ferner bevorzugt kann die Frequenz in Abhängigkeit der Größe der zu erzeugenden Desinfektionsmitteltröpfchen eingestellt werden. Vorzugsweise ist es vorgesehen, die Zerstäubungsmembran mit einer Frequenz im Ultraschallbereich oszillieren zu lassen. Hierdurch können besonders kleine Desinfektionsmitteltröpfchen im Bereich unterhalb von 10 µm erzeugt werden.

Die Vorrichtung weist erfindungsgemäß darüber hinaus eine mit der Aerosolerzeugungseinheit leitungstechnisch verbundenen Ventilationseinheit zum Ansaugen der Umgebungsluft und zum Ausleiten des Aerosols auf. Mittels dieser wird die Raumluft, vorzugsweise die Umgebungsluft, angesaugt und an der Aerosolerzeugungseinheit vorbeibewegt, so dass von der Aerosolerzeugungseinheit erzeugte Desinfektionsmitteltröpfchen in den Luftstrom eingebracht und alsdann zurück in den zu entkeimenden Raum verbracht werden können.

Wie bereits beschrieben, kann dem Desinfektionsmittel während des kontinuierlichen Betriebs Ozon beigemischt werden. Hierzu verfügt die Vorrichtung über eine Ozonerzeugungseinheit. Die Ozonerzeugungseinheit kann vorzugsweise in Form von Ozonkerzen ausgebildet ist. Vorzugsweise sind die Ozonkerzen mit dem Desinfektionsmitteltank zur Einleitung des erzeugten Ozons in das Desinfektionsmittel leitungstechnisch verbunden ist. Neben Ozonkerzen können auch weitere aus dem Stand der Technik bekannte Ozonerzeugungseinheiten verwendet werden. Diese können entweder alleine oder in Kombination miteinander betrieben werden.

Vorzugsweise verfügt die Vorrichtung über eine Fehlerausgabeeinheit. Diese dient der Anzeige von mit dem Betrieb der Vorrichtung insgesamt oder der Einzelkomponenten in Verbindung stehenden Fehlern. Insbesondere können dies Fehler hinsichtlich des Füllstandes im Desinfektionsmitteltank sein. Dieser kann durch einen Defekt der Pumpe, des Füllstandssensors oder ähnlichem verursacht werden. Ferner können Fehler betreffend die Aerosolerzeugungseinheit angezeigt werden. Insbesondere kann ein Membrandefekt angezeigt werden. Auch Fehler im Zusammenhang mit der Ventilationseinheit, insbesondere ein defekter Antrieb oder dergleichen, können angezeigt werden. Insgesamt führt dies zu einer Vereinfachung einer Wartung und gegebenenfalls einer Reparatur der Vorrichtung, da Fehlerquellen erkannt und direkt angezeigt werden können. Vorzugsweise ist die Fehlerausgabeeinheit als LED-Feld ausgebildet. Vorzugsweise ist es vorgesehen, jedem Bauteil eine andersfarbige LED zuzuordnen. Besonders bevorzugt werden Fehler des Desinfektionsmitteltanks mittels einer grünen LED angezeigt. Ferner bevorzugt werden Fehler der Aerosolerzeugungseinheit mittels einer roten LED angezeigt. Ebenfalls bevorzugt werden Fehler der Ventilationseinheit mittels einer gelben LED angezeigt.

Gemäß einer besonders bevorzugten Ausgestaltung der Erfindung ist die Fehlerausgabeeinheit als Displayeinheit ausgebildet. Vorzugsweise handelt es sich um ein Touchdisplay, um unterschiedliche Fehlerstände abzurufen anzuzeigen. Zu diesem Zweck verfügt die Vorrichtung über einen Datenspeicher, in welchem Fehlermeldungen abrufbar abgespeichert werden.

Gemäß einer bevorzugten Ausgestaltung der Erfindung verfügt die Vorrichtung ferner über eine Steuereinrichtung. Diese dient insbesondere der Steuerung der Einzelkomponenten. Insbesondere können damit Flüssigkeitsströme zwischen Vorratsbehälter und Desinfektionsmitteltank gesteuert werden. Ferner ist der Flüssigkeitsstrom vom Desinfektionsmitteltank zur Aerosolerzeugungseinheit durch die Steuereinrichtung steuerbar. Zu diesem Zweck kann die Steuereinrichtung mit Pumpen und/oder Ventilen und/oder sonstigen strömungsregelnden Einrichtungen steuerungstechnisch verbunden sein. Die Bereitstellung einer Steuerungseinrichtung erlaubt in vorteilhafter Weise einen automatisierten Betrieb der erfindungsgemäßen Vorrichtung. Hierdurch können insbesondere der Wartungsaufwand verringert und die Effizienz des Verfahrens insgesamt verbessert werden. Gemäß einer weiteren bevorzugten Ausgestaltung der Erfindung kann die Steuereinrichtung zu Detektion von Fehlerzuständen ausgebildet sein. Zu diesem Zweck ist die Steuerungseinrichtung mit entsprechenden Sensoren verbunden. Diese Sensoren können hierzu bevorzugt in den Einzelkomponenten angeordnet sein. Ferner ist die Steuerungseinrichtung hierzu mit der Fehlerausgabeeinheit verbunden. Vorher detektierte Fehlerzustände können hierdurch an die Fehlerausgabeeinheit gemeldet werden, die diese in einem solchen Fall anzeigt.

Ferner betrifft die Erfindung ein System zur Entkeimung von Räumen oder Bereichen hiervon.

Das System besteht aus einer erfindungsgemäßen Vorrichtung und einem erfindungsgemäßen Desinfektionsmittel. Durch das erfindungsgemäße System wird eine dauerhaft sichere Entkeimung erreicht und eine Wiederverkeimung wirksam vermieden. Die aus dem Stand der Technik unbekannte synergetische Kombination aus erfindungsgemäßer Vorrichtung und erfindungsgemäßem Desinfektionsmittel ermöglichen die kontinuierliche Verfahrensführung des erfindungsgemäßen Verfahrens. Die bevorzugten Merkmale der Einzelkomponenten des erfindungsgemäßen Systems wirken in synergetischer Weise für eine insgesamte weitere Verbesserung des erfindungsgemäßen Systems über die Summe ihrer Einzelteile hinaus.

Die Erfindung betrifft im besonderen ein
Verfahren zur Entkeimung von Räumen oder Bereichen hiervon, bei dem ein Desinfektionsmittel verwendet wird, welches ein Oxidationsmittel und ein Tensid aufweist, bei dem aus dem Desinfektionsmittel ein schwebfähiges Aerosol erzeugt und kontinuierlich in den zu entkeimenden Raum eingebracht wird, bei dem die Gesamtkonzentration des Desinfektionsmittels im Aerosol eingestellt wird, wobei jeweils ein maximaler Grenzwert für die Einzelkonzentration des Oxidationsmittels und des Tensids im Aerosol vorgegeben wird.

Es ist bevorzugt, dass zwischen 0,01 und 0,05 ml Desinfektionsmittel pro 1 m³ Raumluft mittels einer mit einer Ultraschallfrequenz oszillierenden Membran zu Tröpfchen mit einem Durchmesser unterhalb von 10 µm zerstäubt werden.

Es ist bevorzugt, dass das Aerosol aus einem Desinfektionsmittel wenigstens bestehend aus 95 bis 98 Gew.-% vollentsalztem Wasser, 1,5 bis 4,9 Gew.-% H₂O₂ und 0,1 bis 0,5 Gew.-% wenigstens eines Tensids, erzeugt wird, wobei zwischen 0,01 und 0,05 ml Desinfektionsmittel pro 1 m³ Raumluft mittels einer mit einer Ultraschallfrequenz oszillierenden Membran zu Tröpfchen mit einem Durchmesser unterhalb von 5 µm zerstäubt werden.

Es ist ferner bevorzugt, dass vor der Erzeugung des Aerosols Ozon erzeugt und in das Desinfektionsmittel eingeleitet wird.

Die Erfindung betrifft außerdem ein Desinfektionsmittel zum Einsatz erfindungsgemäßen Verfahren, aufweisend ein Oxidationsmittel und ein Tensid.

Es ist hierbei bevorzugt, dass das Desinfektionsmittel als wässrige Desinfektionsmittellösung ausgebildet ist, in der das Oxidationsmittel als H₂O₂ und das Tensid als ein pflanzliches Tensid vorliegt.

Es ist ferner bevorzugt, dass die Desinfektionsmittellösung 95 bis 98 Gew.-% vollentsalztes Wasser, 1,5 bis 4,9 Gew.-% H₂O₂ und 0,1 bis 0,5 Gew.-% wenigstens ein pflanzliches Tensid aufweist.

Es ist darüber hinaus bevorzugt, dass die wässrige Desinfektionsmittellösung weitere pflanzliche Tenside aufweist, wobei alle in der Desinfektionsmittellösung enthaltenen Tenside aus Kokosöl gewinnbar sind.

Es ist weiter bevorzugt, dass die wässrige Desinfektionsmittellösung ein weiteres Oxidationsmittel in Form von Ozon aufweist.

Die Erfindung betrifft außerdem eine Vorrichtung zur Durchführung des erfindungsgemäßen Verfahrens, mit einem Desinfektionsmitteltank zur Bereitstellung eines Desinfektionsmittels, einer mit dem Desinfektionsmitteltank leitungstechnisch verbundenen Aerosolerzeugungseinheit zur Erzeugung eines Aerosols aus dem Desinfektionsmittel und einer mit der Aerosolerzeugungseinheit leitungstechnisch verbundenen Ventilationseinheit zum Ansaugen von Umgebungsluft und zum Ausleiten des Aerosols.

Es ist hierbei bevorzugt, dass die Aerosolerzeugungseinheit eine mit einer Ultraschallfrequenz oszillierbar ausgebildete Zerstäubungsmembran aufweist.

Vorrichtungsseitig ferner bevorzugt ist eine Ozonerzeugungseinheit, welche mit dem Desinfektionsmitteltank zur Einleitung des erzeugten Ozons in das Desinfektionsmittel leitungstechnisch verbunden ist.

Die Erfindung betrifft außerdem ein System zur Entkeimung von Räumen, aufweisend ein erfindungsgemäßes Desinfektionsmittel und eine erfindungsgemäße Vorrichtung.

Neben der Raumentkeimung betrifft ein weiterer wichtiger Teilbereich der Hygiene die gezielte Desinfektion von besonders exponierten Gegenständen oder Körperteilen, insbesondere der Hände und Füße, die regelmäßig in Kontakt mit dem menschlichen Körper gebracht wird. Dies sind in erster Linie medizinische Werkzeuge, wie insbesondere chirurgisches Operationsbesteck oder Dentalwerkzeuge. Die Gefahr einer Infektion bei der Verwendung von nicht ordnungsgemäß desinfizierten Werkzeugen oder Körperteilen ist aufgrund der unmittelbaren Exposition vergleichsweise hoch. In diesem Zusammenhang wirkt sich im Besonderen auch die zunehmende Resistenzbildung der Keime gegenüber gängigen Antibiotika und Desinfektionsmitteln aus. Krankenhäuser sind in dieser Hinsicht besonders gefährdet und daher bemüht, multiresistente Erreger wie MRSA (Methicillin-resistenter Staphylococcus aureus), VRSA (Vancomycin-resistenter Staphylococcus aureus), MRGP (Mehrfach-resistente gram-positive Bakterien) oder MRGN (Mehrfach-resistente gram-negative Bakterien) zu bekämpfen.

Es ist in diesem Zusammenhang die Händedesinfektion mittels flüssiger Desinfektionsmittel bekannt. Desinfektionsmittelspender sollten sich zu diesem Zweck an jedem Ein- und Ausgang hygienischer Bereiche, wie insbesondere Patientenzimmern, befinden. Das medizinische Personal, Patienten und sonstige Besucher sind angehalten sich vor und nach jeder potentiellen Exposition zu desinfizieren. Allerdings bedarf es hierzu einer besonderen Verfahrensweise, mit welcher das nicht-medizinische Personal nicht vertraut ist und das medizinische Personal aufgrund des zeitlichen Aufwands oftmals nur sporadisch einhält. Darüber hinaus bleiben die Hände nach der Desinfektion in der Regel feucht. Oftmals führt dies dazu, dass die Hände mittels unsterilen Hilfsmitteln, wie insbesondere Kleidungsstücken getrocknet werden. Im Ergebnis steht jedenfalls eine unzureichende Desinfektion und ein vergleichsweise hohes Übertragungsrisiko von Infektionen.

Auch die Desinfektion von chirurgischem Operationsbesteck bedarf der Verbesserung. Es ist gängige Praxis, für jede Operation ein eigenes steriles und versiegeltes Besteckset zu verwenden. Nach Öffnen der Verpackung werden die einzelnen Instrumente auf einer Ablage angeordnet, im Falle der Benutzung dem Operateur gereicht, nach der Nutzung wieder auf die Ablage gelegt. Bei einer erneuten Benutzung wird das gleiche Instrument verwendet. Problematisch ist in diesem Zusammenhang, dass die Gefahr einer Kontamination des Bestecks z.B. durch die Luft oder über mangelnde Desinfektion der Ablage mit zunehmender Operationsdauer stetig zunimmt. Auch das Operieren in von Natur aus unsteriler Umgebung, wie insbesondere im Hals-Nasen-Ohren Bereich ist problematisch. Erreger können dabei ungewollt über das Operationsbesteck in noch nicht infizierte Bereiche transportiert werden.

Unglücklicherweise haben sich bislang alle Ansätze, medizinisches Gerät während der Operation zu desinfizieren und damit steril zu halten als unpraktikabel erwiesen.

Es hat sich herausgestellt, dass das vorbeschriebene Desinfektionsmittel nicht nur für die Entkeimung von Räumen geeignet ist, sondern auch für die Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen.

Es wird demgemäß ein Verfahren vorgeschlagen, bei dem ein Desinfektionsmittel verwendet wird, welches ein Oxidationsmittel und ein Tensid aufweist, bei dem aus dem Desinfektionsmittel ein schwebfähiges Aerosol erzeugt und kontinuierlich mit dem zu desinfizierenden Gegenstand und/oder dem Körperteil in Kontakt gebracht wird, wobei jeweils ein maximaler Grenzwert für die Einzelkonzentration des Oxidationsmittels und des Tensids im Aerosol vorgegeben wird. "Kontinuierlich in Kontakt bringen" bedeutet mit Bezug auf die Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen, dass das Aerosol wenigstens für eine der Desinfektion angemessene minimale Zeitspanne ununterbrochen mit dem zu desinfizierenden Gegenstand oder Körperteil kontaktiert wird.

Durch das erfindungsgemäße Verfahren ist es erstmals möglich, medizinische Werkzeuge und Körperteile vollständig und in benutzerfreundlicher Weise zu desinfizieren. Das Aerosol gelangt dabei problemlos auch zu denjenigen Stellen, die z.B. im Rahmen einer gewöhnlichen Händedesinfektion nicht oder nur mit der richtigen Bewegungstechnik der Hände desinfizierbar sind. So ist insbesondere die Handinnenfläche mit flüssigen Desinfektionsmitteln nur unzureichend desinfizierbar. Die Papillarleisten der Handinnenfläche sind in der Regel derart angeordnet, dass jeweils zwischen zwei Papillarleisten eine Furche ausgebildet ist. Der Abstand der Papillarleisten führt dabei zu einer Art Lotuseffekt, wodurch Flüssigkeit aufgrund ihrer Oberflächenspannung nicht in die Furchen eindringen kann. Keime, die sich in diesen Furchen angesammelt haben, können mit herkömmlichen Desinfektionsmitteln folglich nicht oder nur unzureichend bekämpft werden. Das erfindungsgemäße Aerosol dringt jedoch im Gegensatz zu flüssigen Desinfektionsmitteln problemlos in Hautfurchen aller Art ein und bewirkt auch dort eine nahezu vollständige Desinfektion. Darüber hinaus entsteht bei der erfindungsgemäßen Desinfektion mittels des Aerosols kein Feuchtigkeitsfilm auf dem zu desinfizierenden Gegenstand oder Körperteil, wodurch dieser vorteilhafterweise nach abgeschlossener Desinfektion nicht getrocknet werden muss. Hierdurch wird auch das Risiko einer Wiederverkeimung wirkungsvoll minimiert.

Vorzugsweise wird eine erfindungsgemäße Desinfektion in überraschender Weise bereits bei vergleichsweise geringeren Gesamtkonzentrationen erreicht. Prinzipiell ist eine Desinfektion bereits bei Gesamtkonzentration von 0,01 ml bis 0,05 ml Desinfektionsmittel/m³ Raumluft, welche sich bereits bei der Raumentkeimung bewährt hat, erreichbar. Vorzugsweise wird für die Desinfektion jedoch eine höhere Gesamtkonzentration eingestellt. Die Dauer der benötigten Einwirkzeit zur Erreichung einer vollständigen Desinfektion wird hierdurch vorteilhafterweise verkürzt. Insofern ist es bevorzugt, Gesamtkonzentrationen zu verwenden, die für die gewünschte Reduzierung der Einwirkzeit ausreichend und für den Menschen gesundheitlich unbedenklich sind. Bevorzugt werden zur Herstellung des Aerosols daher zwischen 0,1 bis 2,0 ml Desinfektionsmittel pro m³ Luft zu Tröpfchen zerstäubt. Besonders bevorzugt sind Gesamtkonzentrationen zwischen 0,3 bis 1,0 ml/m³, weiter bevorzugt 0,5 bis 0,7 ml/m³. vorgenannte Gesamtkonzentrationen liegen deutlich unterhalb des AGW für Aerosole von 10 ml/m³ und sind insofern als gesundheitlich unbedenklich einzustufen.

Gemäß einem bevorzugten Merkmal der Erfindung wird das Desinfektionsmittel bei der Herstellung des Aerosols auf eine Tröpfchengröße mit einem mittleren Durchmesser unterhalb von 10 µm, vorzugsweise unterhalb von 5 µm zerstäubt. Hierdurch wird einerseits ein schwebfähiges Aerosol erzeugt und andererseits sichergestellt, dass die Gesamtheit des Aerosols auch schwer zugängliche Bereiche, wie insbesondere Haut- oder Materialfurchen, erreichen und in der Folge desinfizieren kann.

Gemäß einer bevorzugten Ausgestaltung der Erfindung wird das Aerosol für eine Mindestdauer mit dem zu desinfizierenden Gegenstand oder Körperteil kontaktiert. Die Mindestdauer ist hierbei die Zeit, die benötigt wird, um eine nahezu vollständige Desinfektion zu erreichen. Diese richtet sich vorrangig nach Art, Größe und Menge der zu desinfizierenden Gegenstände oder Körperteile sowie nach der Gesamtkonzentration des Aerosols. Sofern eine der bevorzugten Gesamtkonzentration des Aerosols eingestellt wird, beträgt die Mindestdauer in Abhängigkeit der übrigen Faktoren zwischen 20 und 60 Sekunden, vorzugsweise zwischen 20 und 30 Sekunden. Nach Erreichen der Mindestdauer sind die Objekte desinfiziert und können aus dem Einwirkbereich des Aerosols entfernt und sogleich bestimmungsgemäß benutzt werden. Alternativ können die Objekte auch über die Mindestdauer hinaus im Einwirkbereich des Aerosols verbleiben. Hierdurch erhalten die Objekte ihren desinfizierten Zustand bei und können bei Bedarf verwendet werden.

In Weiterbildung der vorbeschriebenen Vorrichtung wird zum Zwecke der Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen ferner vorgeschlagen, die Vorrichtung mit einer Applikationseinheit zur Aufnahme der zu desinfizierenden Gegenstände oder Körperteile und Kontaktierung mit dem Aerosol auszurüsten.

Demgemäß ergibt sich eine Vorrichtung mit einem Desinfektionsmitteltank zur Bereitstellung eines Desinfektionsmittels, einer mit dem Desinfektionsmitteltank leitungstechnisch verbundenen Aerosolerzeugungseinheit zur Erzeugung eines Aerosols aus dem Desinfektionsmittel, einer mit der Aerosolerzeugungseinheit leitungstechnisch verbundenen Ventilationseinheit zum Ansaugen von Umgebungsluft und zum Ausleiten des Aerosols aus der Aerosolerzeugungseinheit, und einer leitungstechnisch mit der Aerosolerzeugungseinheit verbundenen Applikationseinheit, welche einen Desinfektionsbereich in Form eines Volumenraumes bereitstellt, wobei der Volumenraum eine erste Öffnung zur Einleitung des Aerosols einerseits und eine zweite Öffnung zum Einbringen und Entfernen des zu desinfizierenden Gegenstandes und/oder Körperteils andererseits aufweist.

Gemäß einem bevorzugten Merkmal der Erfindung ist der Volumenraum in seinen Dimensionen an die zu desinfizierenden Objekte angepasst. Es ist hierbei mit Bezug auf die Desinfektion von Körperteilen bevorzugt, den Volumenraum in seiner räumlichen Ausgestaltung dem zu desinfizierenden Körperteil nachzubilden. Hierbei ist es insbesondere vorgesehen, den Volumenraum in Form einer Hand, der rechten und/oder der linken, auszubilden, in welchen der Benutzer eine oder beide Hände einführen kann. Gleichfalls ist es möglich, bestimmte Abschnitte des Volumenraumes der Form medizinischer Werkzeuge nachzuempfinden. Es ist hierbei insbesondere vorgesehen, innerhalb des Volumenraumes individuelle Abschnitte auszubilden, die hinsichtlich ihrer Form und Anzahl auf ein standardisiertes Set für chirurgisches Operationsbesteck zugeschnitten sind. Es ist hierbei ebenfalls vorgesehen, den Volumenraum auswechselbar innerhalb der Applikationseinheit anzuordnen. Hierdurch ist die Vorrichtung in vorteilhafter Weise für eine Vielzahl von zu desinfizierenden Objekten optimierbar ausgestaltet, wodurch der Desinfektionsvorgang insgesamt verbessert wird.

Gemäß einem bevorzugten Merkmal der Erfindung ist die erste Öffnung als Düse ausgebildet. Hierdurch wird die Einströmgeschwindigkeit des Aerosols in den Desinfektionsbereich erhöht, wodurch die Verteilung des Aerosols im Desinfektionsbereich verbessert wird. Hierdurch wird vorteilhafterweise die Desinfektionswirkung weiter verbessert.

Gemäß einem weiteren bevorzugten Merkmal der Erfindung verfügt der Volumenraum über eine Vielzahl erster Öffnungen. Die ersten Öffnungen können dabei gleichmäßig über Wände des Volumenraumes verteilt angeordnet sein. Gleichmäßig meint hierbei insbesondere, dass unmittelbar benachbarte erste Öffnungen äquidistant zueinander angeordnet sind. Hierdurch wird die gleichmäßige Verteilung des Aerosols im Volumenraum weiter verbessert. Alternativ hierzu sind die ersten Öffnungen heterogen an den Wänden des Volumenraumes angeordnet. Hieraus ergibt sich insbesondere mit der besonderen Ausgestaltung der Form des Volumenraumes die Möglichkeit, eine vergleichsweise große Zahl erster Öffnungen in Bereichen anzuordnen, die Bereichen des zu desinfizierenden Objektes mit erhöhter Kontaminationswahrscheinlichkeit in unmittelbarer Nähe anzuordnen. Hierdurch wird sichergestellt, dass besonders gefährdete Bereiche zuverlässig desinfiziert werden. Insbesondere auch dann, wenn die vorgeschriebene Mindestdauer nicht eingehalten wird. Im Falle der Händedesinfektion könnte es insofern vorgesehen sein, eine vergleichsweise große Zahl erster Öffnungen in der Nähe, vorzugsweise gegenüber, der Handinnenseite anzuordnen. Die dem Handrücken zugewandte Wand des Volumenraumes könnte demgegenüber eine reduzierte Anzahl erster Öffnungen aufweisen.

Gemäß einer bevorzugten Ausgestaltung der Erfindung verfügt die Vorrichtung ferner über eine Steuereinrichtung. Diese dient insbesondere der Steuerung der Einzelkomponenten. Insbesondere können damit Flüssigkeitsströme zwischen Vorratsbehälter und Desinfektionsmitteltank gesteuert werden. Ferner ist der Flüssigkeitsstrom vom Desinfektionsmitteltank zur Aerosolerzeugungseinheit durch die Steuereinrichtung steuerbar. Zu diesem Zweck kann die Steuereinrichtung mit Pumpen und/oder Ventilen und/oder sonstigen strömungsregelnden Einrichtungen steuerungstechnisch verbunden sein. Die Bereitstellung einer Steuerungseinrichtung erlaubt in vorteilhafter Weise einen automatisierten Betrieb der erfindungsgemäßen Vorrichtung. Hierdurch können insbesondere der Wartungsaufwand verringert und die Effizienz des Verfahrens insgesamt verbessert werden. Gemäß einer weiteren bevorzugten Ausgestaltung der Erfindung kann die Steuereinrichtung zu Detektion von Fehlerzuständen ausgebildet sein. Zu diesem Zweck ist die Steuerungseinrichtung mit entsprechenden Sensoren verbunden. Diese Sensoren können hierzu bevorzugt in den Einzelkomponenten angeordnet sein. Ferner ist die Steuerungseinrichtung hierzu mit der vorbeschriebenen Fehlerausgabeeinheit verbunden. Vorher detektierte Fehlerzustände können hierdurch an die Fehlerausgabeeinheit gemeldet werden, die diese in einem solchen Fall anzeigt.

Gemäß einer bevorzugten Ausgestaltung der Erfindung ist der Aerosolstrom von der Aerosolerzeugungseinheit zur Applikationseinheit durch die Steuereinrichtung steuerbar. Hierzu ist die Steuereinrichtung vorzugsweise mit der Ventilationseinheit und der Aerosolerzeugungseinheit steuerungstechnisch verbunden. Hierdurch kann die Strömungsgeschwindigkeit des Aerosols und die Menge an erzeugtem Aerosol automatisch an das zu desinfizierende Objekt angepasst werden. Vorteilhafterweise kann hierdurch die Desinfektionswirkung verbessert und die benötigte Mindestdauer verringert werden.

Gemäß einer bevorzugten Ausgestaltung der Erfindung ist die Steuereinrichtung mit einer Bedieneinheit steuerungstechnisch verbunden. Die Bedieneinheit ist vorzugsweise durch den Benutzer bedienbar ausgebildet. Vorzugsweise ist der Desinfektionsprozess mittels der Bedieneinheit aktivierbar. Sie verfügt zu diesem Zweck über einen benutzerseitig betätigbar ausgebildeten Schalter. Alternativ oder in Kombination hiermit verfügt Die Bedieneinheit vorzugsweise über einen Sensor. Der Sensor dient der automatisierten Aktivierung des Desinfektionsprozesses. Vorzugsweise wird die Desinfektion durch die Steuereinrichtung gestartet, sobald die Sensoren ein zu desinfizierendes Objekt innerhalb eines vorgebbaren Bereiches detektiert haben. Zu diesem Zweck können die Sensoren, als Lichtsensoren, Wärmesensoren und/oder Berührungssensor ausgebildet sein. Vorzugsweise ist wenigstens ein Lichtsensor innerhalb der Applikationseinheit im Bereich der zweiten Öffnung angeordnet, so dass die Einbringung eines zu desinfizierenden Objektes in die Applikationseinheit vom Sensor erfasst wird. Alternativ oder in Kombination hiermit ist es vorzugsweise vorgesehen, einen Berührungssensor, bevorzugt einen Drucksensor, benutzerseitig an der Außenwand der Vorrichtung anzuordnen. Hierdurch wird der Benutzer in die Lage versetzt, die Desinfektion eigenständig aktivieren zu können. Vorzugsweise wird die Desinfektion hierbei mittels des Drucksensors aktiviert. Mittels des Lichtsensors wird überprüft, ob sich zu desinfizierende Objekte im Desinfektionsbereich befinden. Vorzugsweise wird die Desinfektion nur gestartet, wenn sich auch Objekte im Desinfektionsbereich befinden. Befinden sich keine Objekte im Desinfektionsbereich, ist es bevorzugterweise vorgesehen, dass dem Benutzer über die Fehlerausgabeeinheit eine Fehlermeldung ausgegeben wird. Im Falle der Händedesinfektion ist es in diesem bevorzugten Fall vorgesehen, den Drucksensor in einem Bereich der Vorrichtung anzuordnen, der mit der Position der Knie oder der Füße eines durchschnittlichen Benutzers korrespondiert. In diesem Fall kann der Benutzer seine zu desinfizierenden Hände in den Desinfektionsbereich einführen, wobei die Hände vom Lichtsensor erfasst werden und mittels eines Fußes oder eines Knies den Drucksensor betätigen. Im Falle der Fußdesinfektion ist es demgegenüber vorgesehen, den Drucksensor mittels der Hand zu aktivieren und die ordnungsgemäße Positionierung der Füße mittels des Lichtsensors zu detektieren. Die Anordnung der Sensoren ergibt sich entsprechend.

Gemäß einer bevorzugten Ausgestaltung der Erfindung erfasst die Steuereinrichtung den Aktivierungszeitpunkt der Desinfektion sekundengenau, vorzugsweise millisekundengenau, und errechnet mittels einer vorgebbaren Mindestdauer sekundengenau, vorzugsweise millisekundengenau, einen Endzeitpunkt für die Desinfektion. Vorzugsweise sind in der Steuereinrichtung verschiedenen Werte für die zu verwendende Mindestdauer in Abhängigkeit des zu desinfizierenden Objektes hinterlegt. Weiter bevorzugt kann der Benutzer die jeweilige Mindestdauer über die Bedieneinheit abrufen und auswählen. Die Bedieneinheit verfügt zu diesem Zweck vorzugsweise über ein Touchdisplay. Ist der Endzeitpunkt erreicht, wird der Desinfektionsprozess von der Steuereinheit automatisch beendet. Vorzugsweise wird der Benutzer auf das Erreichen des Endzeitpunktes hingewiesen. Vorzugsweise ist die Steuereinrichtung hierzu mit geeigneten Signaleinheiten, wie insbesondere einem Lautsprecher zur Erzeugung eines akustischen Signals und/oder einer LED zur Erzeugung eines visuellen Signals steuerungstechnisch verbunden.

Vorzugsweise kann die erfindungsgemäße Vorrichtung sowohl zur Raumentkeimung als auch zur Desinfektion simultan betrieben werden. Vorzugsweise wird der erzeugte Aerosolstrom hierzu teilweise in den zu entkeimenden Raum eingebracht und teilweise der Applikationseinheit zugeführt. Bevorzugt wird das Aerosol hierbei in der für die Raumentkeimung benötigten Konzentration erzeugt. Vorzugsweise wird das Aerosol anschließend in der Applikationseinheit selbst und/oder in einer zwischengeschalteten Konzentrierungseinheit auf die für die Desinfektion bevorzugte Konzentration aufkonzentriert.

Gemäß einer alternativen Ausgestaltung der Erfindung verfügt die Vorrichtung entweder über zwei separate Aerosolerzeugungseinheiten oder über eine Aerosoleinheit mit leitungstechnisch voneinander getrennten Bereichen zur Erzeugung des Aerosols für die Raumentkeimung einerseits und die Erzeugung des Aerosols für die Desinfektion andererseits. Zu diesem Zweck wird das Desinfektionsmittel teilweise den jeweiligen Aerosolerzeugungseinheiten bzw. den getrennten Bereichen zugeführt.

Sowohl das Verhältnis des Aerosolstromes gemäß der ersten Alternative, als auch die Desinfektionsmittelströme gemäß der zweiten Alternative sind über die Steuereinrichtung einstell- und steuerbar.

Die Erfindung wird nachfolgend anhand eines für den Fachmann nicht beschränkend zu verstehenden Ausführungsbeispiels näher erläutert. Dabei zeigt:
- Fig. 1: eine erfindungsgemäße Vorrichtung zur Durchführung des erfindungsgemäßen Verfahrens gemäß einer ersten Ausführungsform in schematischer Darstellung;
- Fig. 2: eine erfindungsgemäße Vorrichtung zur Durchführung des erfindungsgemäßen Verfahrens gemäß einer zweiten Ausführungsform in schematischer Darstellung.

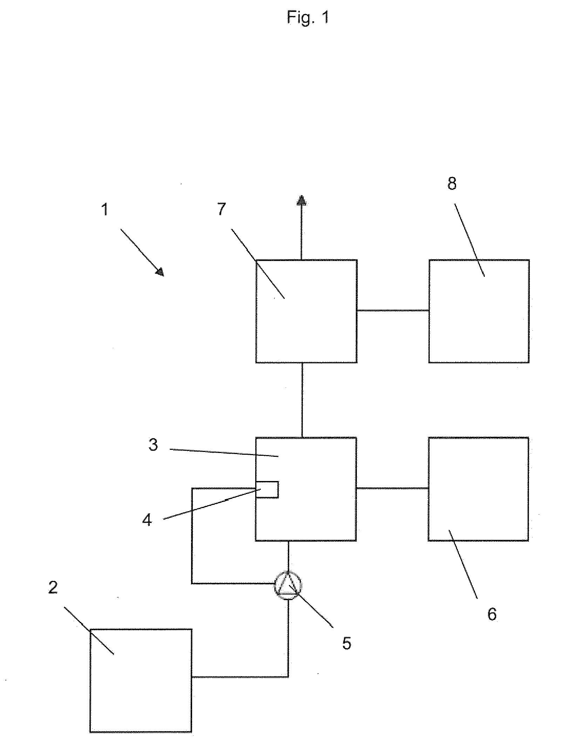

Fig. 1 zeigt eine erfindungsgemäße Vorrichtung 1 mit einem Vorratsbehälter 2. Der Vorratsbehälter 2 ist leitungstechnisch mit dem Desinfektionsmitteltank 3 verbunden. Die Verbindung des Desinfektionsmitteltanks 3 und des Vorratsbehälters 2 ist im vorliegenden Beispiel unter Zwischenschaltung einer Schlauchpumpe 5 realisiert. Der Desinfektionsmitteltank 3 verfügt vorliegend über einen Füllstandssensor 4. Der Füllstandssensor 4 ist steuerungstechnisch mit der Schlauchpumpe 5 verbunden. Auf diesem Wege wird bei Unterschreiten eines vorgebbaren Füllstandes im Desinfektionsmitteltank 3 automatisch Desinfektionsmittel aus dem Vorratsbehälter 2 in den Desinfektionsmitteltank 3 verbracht. Hierdurch wird vorteilhafterweise ein kontinuierlicher Betrieb ermöglicht.

Das Desinfektionsmittel ist in diesem Ausführungsbeispiel als wässrige Lösung ausgebildet. Sie besteht demgemäß aus 97 Gew.% vollentsalztem Wasser, 2,7 Gew.% Wasserstoffperoxid und 0,3 Gew.% Tensiden auf Kokosölbasis. Eine solche Wirkstoffkombination hat sich hinsichtlich der Wirksamkeit gegenüber üblichen Keimen als besonders wirkungsvoll erwiesen, so dass vergleichsweise geringe Konzentrationen zu einer im Wesentlichen vollständigen Entkeimung führen. Darüber hinaus weist ist vorgenannte Desinfektionslösung ein vergleichsweise breites Keimbekämpfungsspektrum auf. Es hat sich herausgestellt, dass es gegen Bakterien, Viren, Sporen und Pilze gleichermaßen wirksam ist. Ferner findet durch die Verwendung von Wasserstoffperoxid eine durch die Bildung von Sauerstoff bedingte Luftauffrischung statt, die von anwesenden Personen als angenehm empfunden wird.

Vorliegend ist der Desinfektionsmitteltank 3 mit einer Ozonerzeugungseinheit 6 leitungstechnisch verbunden. Die Ozonerzeugungseinheit 6 ist vorliegend in Form einer Ozonkerze ausgebildet. Das in dem mittels der Ozonkerze erzeugte Ozon wird in die Desinfektionsmittellösung, eingeleitet. Hierdurch löst sich das gebildete Ozon wenigstens teilweise im Desinfektionsmittel. Das derart modifizierte Desinfektionsmittel wird der Aerosolerzeugungseinheit 7 zugeführt. Die Aerosolerzeugungseinheit 7 verfügt vorliegend über eine Zerstäubungsmembran (nicht gezeigt). Die Zerstäubungsmembran oszilliert mit einer Frequenz im Ultraschallbereich. Hierdurch können Desinfektionsmitteltröpfchen mit einem Durchmesser von 3 µm erzeugt werden. Es hat sich herausgestellt, dass eine solche Tröpfchengröße hinsichtlich der Schwebfähigkeit des Aerosols besonders vorteilhaft ist. Ein derartiges Aerosol erreicht auch Bereiche des zu entkeimenden Raumes, welche vergleichsweise weit von der Vorrichtung 1 entfernt sind, ohne zuvor abzusinken. Durch ein solches Aerosol können ferner raue, poröse, insbesondere textile Oberflächen, welche herkömmlichen Desinfektionsverfahren nicht zugänglich sind, besonders wirkungsvoll entkeimt werden. Dies ist insbesondere der in der geringen Tröpfchengröße liegenden, vorteilhaften Materialgängigkeit der Aerosoltröpfchen geschuldet.

Die Aerosolerzeugungseinheit 7 ist mit einer Ventilationseinheit 8 leitungstechnisch verbunden. Die Ventilationseinheit 8 saugt eine die Vorrichtung umgebende Raumluft zum Zwecke der Aerosolerzeugung an. Die Raumluft wird an der Zerstäubungsmembran vorbeibewegt, und dient somit als Trägermedium für das Desinfektionsmittel. Die an der Zerstäubungsmembran erzeugten Mikrotröpfchen werden zu diesem Zweck in den Luftstrom eingebracht. Alsdann wird das erzeugte Aerosol aus der Vorrichtung ausgeleitet. Die Ausleitung kann erfindungsgemäß entweder unmittelbar in den zu entkeimenden Raum erfolgen. Hierzu kann die Vorrichtung 1 in dem zu entkeimenden Raum bzw. dem zu entkeimenden Raumbereich aufgestellt werden. In diesem Fall ist die Vorrichtung 1 mobil ausgebildet, um verschiedene Räume oder Raumbereiche je nach Wunsch oder Notwendigkeit zu entkeimen. Zu diesem Zweck weist die Vorrichtung 1 Rollen auf.

Alternativ kann das Aerosol mittelbar in den zu entkeimenden Raum eingebracht werden. Hierzu ist die Vorrichtung 1 in einem bereits bestehenden Belüftungssystem eines Gebäudes, welches eine Vielzahl von Räumen miteinander verbindet angeordnet, um möglichste viele Räume gleichermaßen zu entkeimen. Der in einem solchen Belüftungssystem vorliegende Luftstrom umspült dabei die Vorrichtung. Er dient hierbei vorteilhafterweise als Trägermedium für das Desinfektionsmittel im Aerosol.

Vorliegend verfügt die Vorrichtung über eine nicht dargestellte Fehlerausgabeeinheit. Die Fehlerausgabeeinheit ist vorliegend als LED-Feld ausgebildet. Fehler des Desinfektionsmitteltanks werden mittels einer grünen LED angezeigt. Fehler der Aerosolerzeugungseinheit werden mittels einer roten LED angezeigt. Fehler der Ventilationseinheit werden mittels einer gelben LED angezeigt. Vorteilhafterweise führt dies insgesamt zu einer Vereinfachung der Wartung und gegebenenfalls der Reparatur der Vorrichtung 1, da Fehlerquellen erkannt und direkt angezeigt werden können.

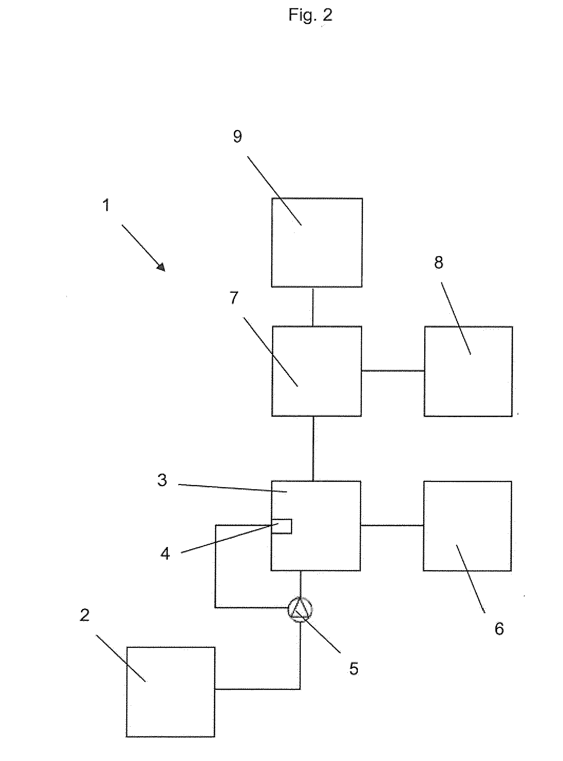

Fig. 2 zeigt eine erfindungsgemäße Vorrichtung 1 mit einem Vorratsbehälter 2. Der Vorratsbehälter 2 ist leitungstechnisch mit dem Desinfektionsmitteltank 3 verbunden. Die Verbindung des Desinfektionsmitteltanks 3 und des Vorratsbehälters 2 ist im vorliegenden Beispiel unter Zwischenschaltung einer Schlauchpumpe 5 realisiert. Der Desinfektionsmitteltank 3 verfügt vorliegend über einen Füllstandssensor 4. Der Füllstandssensor 4 ist steuerungstechnisch mit der Schlauchpumpe 5 verbunden. Auf diesem Wege wird bei Unterschreiten eines vorgebbaren Füllstandes im Desinfektionsmitteltank 3 automatisch Desinfektionsmittel aus dem Vorratsbehälter 2 in den Desinfektionsmitteltank 3 verbracht. Hierdurch wird vorteilhafterweise ein kontinuierlicher Betrieb ermöglicht.

Das Desinfektionsmittel ist in diesem Ausführungsbeispiel als wässrige Lösung ausgebildet. Sie besteht demgemäß aus 97 Gew.% vollentsalztem Wasser, 2,7 Gew.% Wasserstoffperoxid und 0,3 Gew.% Tensiden auf Kokosölbasis. Eine solche Wirkstoffkombination hat sich hinsichtlich der Wirksamkeit gegenüber üblichen Keimen als besonders wirkungsvoll erwiesen, so dass vergleichsweise geringe Konzentrationen zu einer im Wesentlichen vollständigen Entkeimung führen. Darüber hinaus weist ist vorgenannte Desinfektionslösung ein vergleichsweise breites Keimbekämpfungsspektrum auf. Es hat sich herausgestellt, dass es gegen Bakterien, Viren, Sporen und Pilze gleichermaßen wirksam ist und in der erfindungsgemäßen Konzentration für die Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen geeignet ist.

Vorliegend ist der Desinfektionsmitteltank 3 mit einer Ozonerzeugungseinheit 6 leitungstechnisch verbunden. Die Ozonerzeugungseinheit 6 ist vorliegend in Form einer Ozonkerze ausgebildet. Das in dem mittels der Ozonkerze erzeugte Ozon wird in die Desinfektionsmittellösung, eingeleitet. Hierdurch löst sich das gebildete Ozon wenigstens teilweise im Desinfektionsmittel. Das derart modifizierte Desinfektionsmittel wird der Aerosolerzeugungseinheit 7 zugeführt. Die Aerosolerzeugungseinheit 7 verfügt vorliegend über eine Zerstäubungsmembran (nicht gezeigt). Die Zerstäubungsmembran oszilliert mit einer Frequenz im Ultraschallbereich. Hierdurch können Desinfektionsmitteltröpfchen mit einem Durchmesser von 3 µm erzeugt werden. Es hat sich herausgestellt, dass eine solche Tröpfchengröße hinsichtlich der Schwebfähigkeit des Aerosols sowie dessen Fähigkeit zur Desinfektion von schwer zugänglichen Bereichen besonders vorteilhaft ist. Ein derartiges Aerosol erreicht nämlich auch Bereiche des zu desinfizierenden Objektes, welche aufgrund ihrer geometrischen Ausgestaltung vergleichsweise schwierig zu desinfizieren sind. Durch ein solches Aerosol können insbesondere raue, poröse, insbesondere textile Oberflächen und die menschliche Haut, insbesondere die der Hand- und Fußinnenflächen, welche herkömmlichen Desinfektionsverfahren nicht zugänglich sind, besonders wirkungsvoll desinfiziert werden. Dies ist insbesondere der in der geringen Tröpfchengröße liegenden, vorteilhaften Materialgängigkeit der Aerosoltröpfchen geschuldet.

Die Aerosolerzeugungseinheit 7 ist mit einer Ventilationseinheit 8 leitungstechnisch verbunden. Die Ventilationseinheit 8 saugt eine die Vorrichtung umgebende Raumluft zum Zwecke der Aerosolerzeugung an. Die Raumluft wird an der Zerstäubungsmembran vorbeibewegt, und dient somit als Trägermedium für das Desinfektionsmittel. Die an der Zerstäubungsmembran erzeugten Mikrotröpfchen werden zu diesem Zweck in den Luftstrom eingebracht. Das Aerosol wird vorliegend mit einer Konzentration von 0,6 ml/m³ erzeugt. Alsdann wird das erzeugte Aerosol einer Applikationseinheit 9 zugeleitet. Zu diesem Zweck, sind die Aerosolerzeugungseinheit 7 und die Applikationseinheit 9 leitungstechnisch miteinander verbunden. Die Applikationseinheit 9 stellt einen Desinfektionsbereich in Form eines Volumenraumes bereit. Der Desinfektionsbereich verfügt über eine Vielzahl erster Öffnungen zur Einleitung des Aerosols in den Desinfektionsbereich, die gleichmäßig über dessen Innenseite angeordnet sind. Ferner verfügt der Desinfektionsbereich über eine zweite Öffnung zur Einbringung der zu desinfizierenden Objekte in den Desinfektionsbereich.

In diesem Ausführungsbeispiel ist die Applikationseinheit 9 als Händedesinfektionseinheit ausgebildet. Zu diesem Zweck verfügt die Vorrichtung 1 über eine nicht dargestellte Steuereinrichtung, die mit einer Bedieneinheit zur Aktivierung des Desinfektionsvorganges steuertechnisch verbunden ist. Die Bedieneinheit ist vorliegend mit einem Drucksensor und einem Lichtsensor ausgerüstet. Der Drucksensor ist an der bei bestimmungsgemäßem Gebrauch der Vorrichtung benutzerseitigen Außenwand der Vorrichtung in Kniehöhe eines durchschnittlichen Benutzers angeordnet. Bei Betätigung des Drucksensors wird die Desinfektion, vorzugsweise die Einleitung des Aerosols in den Desinfektionsmittelbereich, gestartet, sofern der Lichtsensor die ordnungsgemäße Positionierung der Hände innerhalb des Desinfektionsbereiches detektiert. Die Steuereinrichtung erfasst nun den Startzeitpunkt. Vorliegend ist eine Desinfektionsdauer von 20 Sekunden in der Steuereinrichtung hinterlegt. Auf Basis der hinterlegten Desinfektionsdauer errechnet die Steuereinrichtung einen Endzeitpunkt für die Desinfektion. Ist dieser erreicht, wird die Desinfektion, vorzugsweise die Einleitung des Aerosols in den Desinfektionsbereich gestoppt. Ferner wird der Benutzer auf die Beendigung der Desinfektion mittels eines geeigneten Signals, vorliegend ein akustisches Signal, hingewiesen.

Vorliegend verfügt die Vorrichtung über eine nicht dargestellte Fehlerausgabeeinheit. Die Fehlerausgabeeinheit ist vorliegend als LED-Feld ausgebildet. Fehler des Desinfektionsmitteltanks werden mittels einer grünen LED angezeigt. Fehler der Aerosolerzeugungseinheit werden mittels einer roten LED angezeigt. Fehler der Ventilationseinheit werden mittels einer gelben LED angezeigt. Vorteilhafterweise führt dies insgesamt zu einer Vereinfachung der Wartung und gegebenenfalls der Reparatur der Vorrichtung 1, da Fehlerquellen erkannt und direkt angezeigt werden können.

### Bezugszeichenliste

- 1: Vorrichtung
- 2: Vorratsbehälter
- 3: Desinfektionsmitteltank
- 4: Flüssigkeitsmesser
- 5: Schlauchpumpe
- 6: Ozonkerze
- 7: Aerosolerzeugungseinheit
- 8: Ventilationseinheit
- 9: Applikationseinheit

## Patentansprüche

1. Verfahren zur Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen, bei dem ein Desinfektionsmittel verwendet wird, welches Wasserstoffperoxid und ein pflanzliches Tensid aufweist, bei dem aus dem Desinfektionsmittel ein schwebfähiges Aerosol erzeugt und kontinuierlich mit dem zu desinfizierenden Gegenstand und/oder dem Körperteil in Kontakt gebracht wird, wobei jeweils ein maximaler Grenzwert für die Einzelkonzentration des Oxidationsmittels und des Tensids im Aerosol vorgegeben wird.

2. Verfahren nach Anspruch 1, **dadurch gekennzeichnet, dass** bei der Aerosolerzeugung 0,1 bis 1,0 ml, vorzugsweise 0,5 bis 0,7 ml, Desinfektionsmittel pro m³ Luft zerstäubt werden.

3. Verfahren nach Anspruch 1 oder 2, **dadurch gekennzeichnet, dass** bei der Aerosolerzeugung das Desinfektionsmittel zu Tröpfchen mit einer Größe unterhalb von 10 µm, vorzugsweise unterhalb von 5 µm zerstäubt werden.

4. Verfahren nach einem der Ansprüche 1 bis 3, **dadurch gekennzeichnet, dass** das Aerosol für eine Mindestdauer von 20 bis 60 Sekunden kontinuierlich mit dem zu desinfizierenden Gegenstand und/oder dem Körperteil in Kontakt gebracht wird.

5. Aerosol zur Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen, bestehend aus einem Desinfektionsmittels, aufweisend Wasserstoffperoxid und ein pflanzliches Tensid, und Luft, wobei das Desinfektionsmittel in Form von Tröpfchen mit einem Durchmesser unterhalb von 10 µm und in einer Volumenkonzentration von 0,1 bis 1,0 ml pro m³ Luft im Aerosol vorliegt.

6. Aerosol nach Anspruch 5, **dadurch gekennzeichnet, dass** das pflanzliche Tensid ein aus Kokosöl gewinnbares Tensid ist.

7. Vorrichtung zur Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen, mit einem Desinfektionsmitteltank zur Bereitstellung eines Desinfektionsmittels, einer mit dem Desinfektionsmitteltank leitungstechnisch verbundenen Aerosolerzeugungseinheit zur Erzeugung eines Aerosols aus dem Desinfektionsmittel, einer mit der Aerosolerzeugungseinheit leitungstechnisch verbundenen Ventilationseinheit zum Ansaugen von Umgebungsluft und zum Ausleiten des Aerosols aus der Aerosolerzeugungseinheit, und einer leitungstechnisch mit der Aerosolerzeugungseinheit verbundenen Applikationseinheit, welche einen Desinfektionsbereich in Form eines Volumenraumes bereitstellt, wobei der Volumenraum eine erste Öffnung zur Einleitung des Aerosols einerseits und eine zweite Öffnung zum Einbringen und Entfernen des zu desinfizierenden Gegenstandes und/oder Körperteils andererseits aufweist.

8. Vorrichtung nach Anspruch 7, **dadurch gekennzeichnet, dass** die Aerosolerzeugungseinheit eine mit einer Ultraschallfrequenz oszillierbar ausgebildete Zerstäubungsmembran aufweist.

9. Vorrichtung nach einem der Ansprüche 7 oder 8, **dadurch gekennzeichnet, dass** der Volumenraum über eine Vielzahl erster Öffnungen verfügt.

10. Vorrichtung nach Anspruch 9, **dadurch gekennzeichnet, dass** die Vielzahl erster Öffnungen gleichmäßig über die Wände des Volumenraumes verteilt angeordnet sind.

11. Vorrichtung nach einem der Ansprüche 7 bis 10, **gekennzeichnet durch** eine Bedieneinheit, welche mit einer Steuereinrichtung steuerungstechnisch verbunden ist und einen Drucksensor und/oder einen Lichtsensor aufweist.

12. Verwendung eines Aerosols nach einem der Ansprüche 5 oder 6 zur Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen.

13. Verwendung einer Vorrichtung nach einem der Ansprüche 7 bis 11 zur Desinfektion von medizinischen Werkzeugen, vorzugsweise chirurgischem Operationsbesteck, zahnmedizinischen Werkzeugen und/oder von bedeckten und unbedeckten Körperteilen, wie insbesondere Händen und Füßen.
